# EUROPEAN PATENT SPECIFICATION

(11) **EP 2 334 676 B1**
(45) Date of publication and mention of the grant of the patent: **06.07.2016**
(21) Application number: 09792255.3
(22) Date of filing: 04.09.2009
(51) Int. Cl.: C07D 471/04, A61K 31/4745, A61P 29/00

(54) **SUBSTITUTED 3-AMINO-1-OXO OR THIOXO-1,2,5,6,7,8-HEXAHYDRO-2,7-NAPHTHYRIDINE-4-CARBONITRILES AS SELECTIVE ALPHA 2B ANTAGONISTS**
SUBSTITUIERTE 3-AMINO-1-OXO ODER THIOXO-1,2,5,6,7,8-HEXAHYDRO-2,7-NAPHTHYRIDIN-4-CARBONITRILE ALS SELECTIVE ALPHA 2B ANTAGONISTEN
3-AMINO-1-OXO OU THIOXO-1,2,5,6,7,8-HEXAHYDRO-2,7-NAPHTYRIDINE-4-CARBONITRILES SUBSTITUÉS EN TANT QU'ANTAGONISTES SÉLECTIFS D'ALPHA-2B

(30) Priority: 17.09.2008 US 97624 P
(43) Date of publication of application: 22.06.2011
(73) Proprietor: ALLERGAN, INC., Irvine, CA 92612 (US)
(72) Inventor: CAPPIELLO, John, R., Irvine CA 92612 (US); CHOW, Ken, Newport Coast CA 92657 (US); HEIDELBAUGH, Todd, M., Fountain Valley CA 92708 (US); TAKEUCHI, Janet, A., Anaheim CA 92808 (US); GARST, Michael, E., Newport Beach CA 92660 (US); GIL, Daniel, W., Corona Del Mar CA 92625 (US); KEDZIE, Karen, M., Rancho Santa Margarita CA 92688 (US); DONELLO, John, E., Dana Point CA 92629 (US)
(74) Representative: Hoffmann Eitle
(86) International application number: PCT/US2009/055979
(87) International publication number: WO 2010/033393

(56) References cited:
- WO-A-2008/005910
- DATABASE REGISTRY CHEMICAL ABSTRACTS SERVICE, COLUMBUS, OHIO, US; 7 January 2002 (2002-01-07), XP002554305 retrieved from STN Database accession no. 380586-98-1
- DATABASE REGISTRY CHEMICAL ABSTRACTS SERVICE, COLUMBUS, OHIO, US; 30 November 2001 (2001-11-30), XP002554306 retrieved from STN Database accession no. 372497-57-9
- DATABASE REGISTRY CHEMICAL ABSTRACTS SERVICE, COLUMBUS, OHIO, US; 28 November 2001 (2001-11-28), XP002554307 retrieved from STN Database accession no. 372088-31-8
- DATABASE REGISTRY CHEMICAL ABSTRACTS SERVICE, COLUMBUS, OHIO, US; 4 December 2001 (2001-12-04), XP002554308 retrieved from STN Database accession no. 373375-36-1
- DATABASE CAPLUS CHEMICAL ABSTRACTS SERVICE, COLUMBUS, OHIO, US; XP002554309 retrieved from STN Database accession no. 1989:23754 & E.G. PARONIKYAN ET AL: ARMYANSKII KHIMICHESKII ZHURNAL, vol. 40, no. 9, 1987, pages 587-593,

## Description

### Field of the Invention

This invention relates to pharmaceutical compositions comprising substituted 3-amino-2,7-dimethyl-1-oxo or thioxo-1,2,5,6,7,8-hexahydro-2,7-naphthyridine-4-carbonitrile ring systems. These compounds are subtype selective antagonists for the alpha 2B receptor and have no or weak antagonist activity at the other alpha adrenergic receptors. These compounds are useful as tool compounds for developing compounds useful in treating diseases that include but are not limited to chronic pain, visceral pain, corneal pain, neuropathic pain, glaucoma, ischemic neuropathies and other neurodegenerative diseases. These compounds are also useful as compounds for treating myocardial infarction and preventing acute coronary events.

### Description of the Related Art

It is noted that in the discussion of the prior art, below, various designations for the adrenergic receptor subtypes are used interchangeably. That is, for the 1A subtype of the alpha adrenergic receptor the designation may be alpha.sub.1A or α_{1A} and the 2B subtype of the alpha adrenergic receptor may be alpha.sub.2B or α_{2B}, etc.

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into alpha1, alpha2, beta.sub.1 and beta.sub.2 subtypes. Functional differences between alpha.sub.1 and alpha.sub.2 receptors have been recognized, and compounds which exhibit selective binding between these two subtypes have been developed. Thus, in published international patent application WO 92/0073, the selective ability of the R(+) enantiomer of terazosin to selectively bind to adrenergic receptors of the alpha.sub.1 subtype was reported. The alpha.sub.1/alpha.sub.2 selectivity of this compound was disclosed as being significant because agonist stimulation of the alpha.sub.2 receptors was said to inhibit secretion of epinephrine and norepinephrine, while antagonism of the alpha.sub.2 receptor was said to increase secretion of these hormones. Thus, the use of non-selective alpha-adrenergic blockers, such as phenoxybenzamine and phentolamine, was said to be limited by their alpha.sub.2 adrenergic receptor mediated induction of increased plasma catecholamine concentration and the attendant physiological sequelae (increased heart rate and smooth muscle contraction).

For a further general background on the .alpha.-adrenergic receptors, the reader's attention is directed to Robert R. Ruffolo, Jr., .alpha.-Adrenoreceptors: Molecular Biology, Biochemistry and Pharmacology, (Progress in Basic and Clinical Pharmacology series, Karger, 1991), wherein the basis of alpha.sub.1/alpha.sub.2 subclassification, the molecular biology, signal transduction, agonist structure-activity relationships, receptor functions, and therapeutic applications for compounds exhibiting alpha-adrenergic receptor affinity is explored.

The cloning, sequencing and expression of alpha receptor subtypes from animal tissues has led to the subclassification of the alpha.sub.1 adrenoreceptors into alpha.sub.1A, alpha.sub.1B, and alpha.sub.1D. Similarly, the alpha.sub.2 adrenoreceptors have also been classified alpha.sub.2A, alpha.sub.2B, and alpha.sub.2C receptors. Each alpha.sub.2 receptor subtype appears to exhibit its own pharmacological and tissue specificities. Compounds having a degree of specificity for one or more of these subtypes may be more specific therapeutic agents for a given indication than an alpha.sub.2 receptor pan-agonist (such as the drug clonidine) or a pan-antagonist.

Among other indications, such as the treatment of glaucoma, hypertension, sexual dysfunction, and depression, certain compounds having alpha 2 adrenergic receptor agonist activity are known analgesics. However, many compounds having such activity do not provide the activity and specificity desirable when treating disorders modulated by alpha-2 adrenoreceptors. For example, many compounds found to be effective agents in the treatment of pain are frequently found to have undesirable side effects, such as causing hypotension and sedation at systemically effective doses.

Thus, there is a need for new drugs that provide relief from pain without causing these undesirable side effects. Additionally, there is a need for agents which display activity against pain, particularly chronic pain, such as chronic neuropathic and visceral pain.

### Summary of the Invention

The present invention is defined by the claims and in one aspect relates to 2,3-diamino-6,6,7-trimethyl-1-thioxo-1,2,5,6,7,8-hexahydro-2,7-naphthyridine-4-carbonitri le for use in a method of therapy.

The invention also relates to compounds represented by the following formula, for use in a method of therapy: 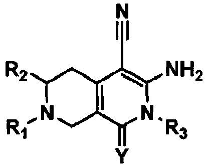 wherein Y is O or S; and R₁ R₂ and R₃ are H, hydrocarbyl, substituted hydrocarbyl or amino.

The invention also relates to compounds represented by the following formula, for use in a method of therapy: 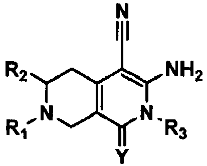 wherein Y is O or S; and wherein R₁, R₂ and R₃ are selected from the group consisting of H, alkyl, aryl and NR₄R₄, wherein R₄ is alkyl.

The invention further relates to compounds above for use in a method of treating pain, glaucoma, ischemic neuropathy, neurodegenerative disease, myocardial infarction or a method of preventing acute coronary events. In a preferred embodiment the invention relates to these compounds for use in a method of treating chronic pain, visceral pain, corneal pain, or neuropathic pain.

### Description of the Drawing Figure

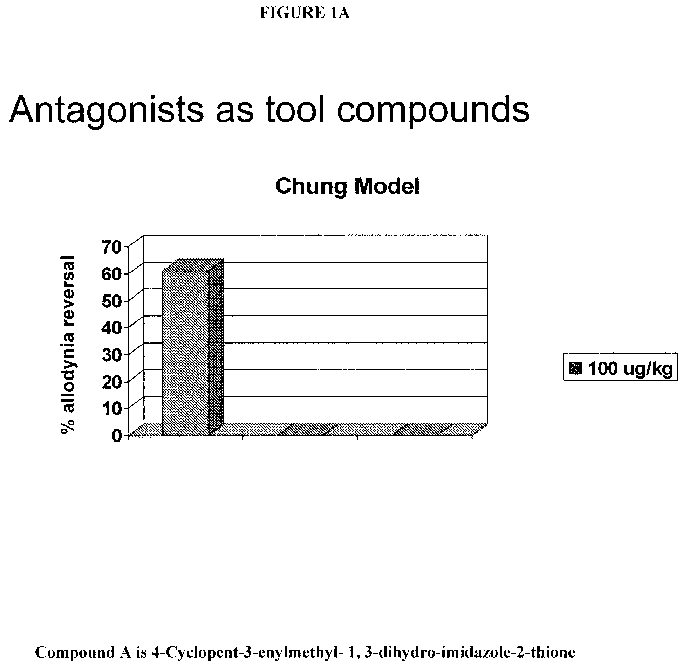

Figure 1A shows the effect of one of the alpha adrenergic receptor antagonists of the invention in a model of allodynic pain.

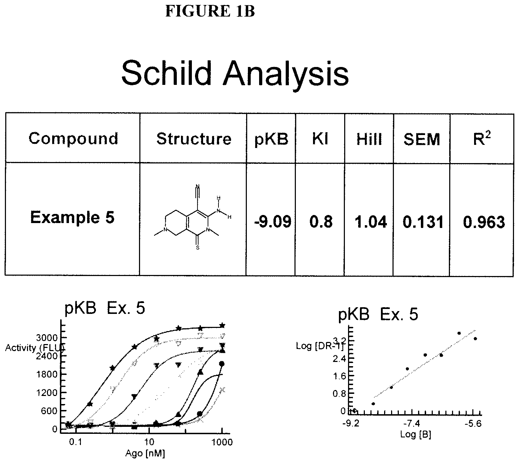

Figure 1B shows the values of one of the alpha adrenergic receptor antagonists of the invention in a Schild Analysis.

### Detailed Description of the Invention

The present invention utilizes compounds which are subtype selective antagonist for the alpha 2B adrenergic receptor and have no or weak antagonist activity at the other alpha adrenergic receptors. These compounds are useful as tool compounds for developing compounds useful in treating diseases that include but not limited to chronic pain, visceral pain, corneal pain, neuropathic pain, glaucoma, ischemic neuropathies and other neurodegenerative diseases and conditions. These compounds are also useful as compounds for treating myocardial infarction and preventing acute coronary events. The compounds for use in this invention are defined in the appended claims and include 2,3-diamino-6,6,7-trimethyl-1-thioxo-1,2,5,6,7,8-hexahydro-2,7-naphthyridine-4-carbonitrile as well as those selected from the group of compounds represented by the formula

### Naphthyridines:

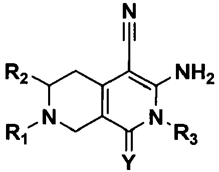

wherein Y is O or S, preferably S; R₁, R₂ and R₃ are H, hydrocarbyl, substituted hydrocarbyl or amino and R₄ is amino and preferably R₁, R₂ and R₃ are selected from the group consisting of H, alkyl, aryl (as defined below) and NR₄R₄, wherein R₄ is alkyl, and more preferably R₁, R₂ and R₃ are H or C₁ to C₄ alkyl and R₄ is C₁ to C₄ alkyl.

The compounds for use in this invention are broadly described as 3-amino-1-oxo or thioxo-1,2,5,6,7,8-hexahydro-2,7-naphthyridine-4-carbonitriles and 2 and/or 6 and/or 7-substituted derivatives thereof, wherein the substituent comprises an alkyl radical, including an alkoxy or an alkaryl radical, or an aryl radical, including an aryloxy or arylalkyloxy radical, wherein said aryl is a carbocyclic or heterocyclic aryl radical, or another radical having a heteroatom selected from the group consisting of halogen, nitrogen oxygen, sulfur and phosphorus, e.g. a fluoro, chloro, nitro, amino, hydroxyl, etc. and pharmaceutically acceptable salts thereof.

In particular, said compounds are 3-amino-1-oxo or thioxo-1,2,5,6,7,8-hexahydro-2,7-naphthyridine-4-carbonitriles and 2-lower alkyl amino and/or 6-lower alkyl and/or 7-lower alkyl-substituted derivatives thereof.

Unless otherwise indicated, the following terms as used throughout this specification have the following meanings:
"Me" refers to methyl.
"Et" refers to ethyl.
"tBu" refers to t-butyl.
"iPr" refers to i-propyl.
"Ph" refers to phenyl.
"boC" refers to tBuOcarbonyl
"rt" refers to room temperature
"ppt" refers to precipitate
"DMSO" refers to dimethylsulfoxide
"DBU" refers to 1,8-diazabicyclo[5.4.0]undec-7-ene
"TFA" refers to trifluoroacetic acid
"Pharmaceutically acceptable salt" refers to those salts which retain the biological effectiveness and properties of the free bases and which are obtained by reaction with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like.
"Alkyl" refers to a straight-chain, branched or cyclic saturated aliphatic hydrocarbon. Preferably, the alkyl group has 1 to 12 carbons. More preferably, it is a lower alkyl of from 1 to 7 carbons, most preferably 1 to 4 carbons. Typical alkyl groups include methyl, ethyl, propyl, isopropyl, butyl, isobutyl, tertiary butyl, pentyl, hexyl and the like. The alkyl group may be optionally substituted with one or more substituents are selected from the group consisting of hydroxyl, cyano, alkoxy, =O, =S, NO₂, halogen, dimethyl amino and SH.
"Alkoxy" refers to an "O-alkyl" group.
"Aryl" refers to an aromatic group which has at least one ring having a conjugated pi electron system and includes carbocyclic aryl, heterocyclic aryl and biaryl groups. The aryl group may be optionally substituted with one or more substituents selected from the group consisting of halogen, trihalomethyl, hydroxyl, SH, OH, NO₂, amine, thioether, cyano, alkoxy, alkyl, and amino.
"Alkaryl" refers to an alkyl that is covalently joined to an aryl group. Preferably, the alkyl is a lower alkyl.
"Aryloxy" refers to an "O-aryl" group.
"Arylalkyloxy" refers to an "O-alkaryl" group.
"Carbocyclic" refers to cyclic saturated or unsaturated aliphatic hydrocarbon and aryl hydrocarbon groups wherein the ring atoms are exclusively carbons, and comprises from 6 to 20 carbon atoms, including said ring atoms.
"Carbocyclic aryl" refers to an aryl group wherein the ring atoms are carbon.
"Heterocyclic" refers to cyclic groups wherein the ring atoms comprise carbon atoms and at least one oxygen, nitrogen, and/or sulfur atom and may be saturated, unsaturated, i.e. have one or more double bonds, or aryl, and comprises up to 20 carbon atoms and from 1 to 5 of the above heteroatoms.
"Heterocyclic aryl" refers to an aryl group having from 1 to 3 heteroatoms as ring atoms, the remainder of the ring atoms being carbon. Heteroatoms include oxygen, sulfur, and nitrogen.
"Hydrocarbyl" refers to a hydrocarbon radical having only carbon and hydrogen atoms. Preferably, the hydrocarbyl radical has from 1 to 20 carbon atoms, more preferably from 1 to 12 carbon atoms and most preferably from 1 to 7 carbon atoms.
"Substituted hydrocarbyl" refers to a hydrocarbyl radical wherein one or more, but not all, of the hydrogen and/or the carbon atoms are replaced by a halogen, nitrogen, oxygen, sulfur or phosphorus atom or a radical including a halogen, nitrogen, oxygen, sulfur or phosphorus atom, e.g. fluoro, chloro, cyano, nitro, hydroxyl, phosphate, thiol, etc.
"Amine" or "amino" refers to a --N(R")R"' group, wherein R" and R'" are independently selected from the group consisting of alkyl, aryl, and alkylaryl.

The preferred compounds for use in this invention are
3-amino-2,7-dimethyl-1-thioxo-1,2,5,6,7,8-hexahydro-2,7-naphthyridine-4-carbonitrile
2,3-diamino-7-methyl-l-thioxo-1,2,5,6,7,8-hexahydro-2,7-naphthyridine-4-carbonitrile
2,3-diamino-6,6,7-trimethyl-1-thioxo-1,2,5,6,7,8-hexahydro-2,7-naphthyridine-4-carbonitrile
3-amino-2-methyl-1-thioxo-1,2,5,6,7,8-hexahydro-2,7-naphthyridine-4-carbonitrile
3-amino-2-ethyl-7-methyl-1-thioxo-1,2,5,6,7,8-hexahydro-2,7-naphthyridine-4-carbonitrile
3-amino-2,7-dimethyl-1-oxo-1,2,5,6,7,8-hexahydro-2,7-naphthyridine-4-carbonitrile

It is noted that the names of the compounds for use in this invention are named with a different system in the Examples, below. Thus, for example 3-amino-2,7-dimethyl-1-thioxo-1,2,5,6,7,8-hexahydro-2,7-naphthyridine-4-carbonitrile is designated as 3-Amino-4-cyano-2,7-dimethyl-(1,2,5,6,7,8-hexahydro-2,7-napthyridine-1-thione) in Example 5. Both compounds names designate the same compound as is apparent from the structure of the compound of Example 5 shown below.

The biological properties of these compounds are summarized in Table 1, below.

**TABLE 1**

| | **IC₅₀ (nM) (% Antagonism)** | | | | |
|---|---|---|---|---|---|
| | **Alpha 1A** | **Alpha 1B** | **Alpha 2A** | **Alpha 2B** | **Alpha 2C** |
| 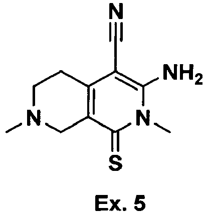 | nd (64) | >8300 (0) | 1992 (69) | 10 (95) | nd (25) |
| 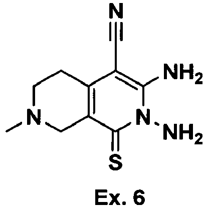 | >7500 (19) | >7500 (19) | nd (36) | 204 (112) | 1407 (29) |
| 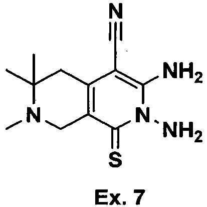 | 382 (98) | >7500 (16) | >7500 (0) | 29 (97) | nd (17) |
| 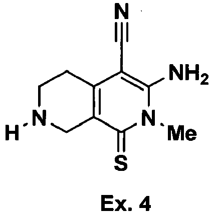 | | | | 342 (93) | |
| 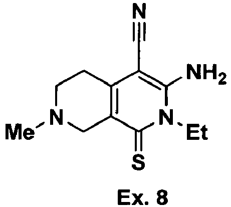 | | | | 473 (95) | |
| 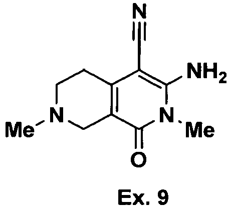 | | | | 1174 (88) | |

The compounds for use in this invention may be prepared as follows:

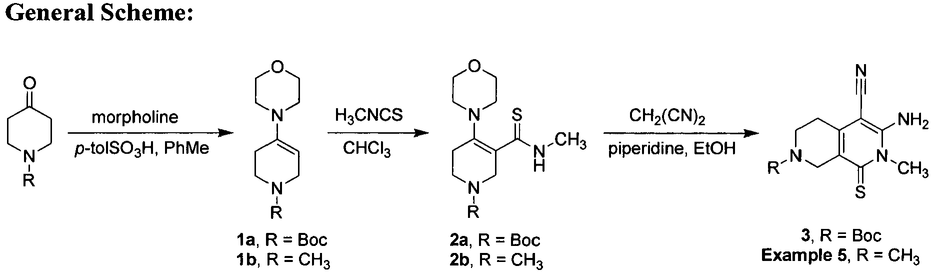

Proton NMR spectra were measured at 60 or 300 MHz on Varian T-60 or Varian Inova 300 spectrometers. Chemical shifts are expressed in ppm. Mass spectra were recorded on an Agilent 1100 SL series LC/MSD spectrometer using electrospray (ESI) or chemical (APCI) ionization. High pressure liquid chromatography analyses were performed using an Agilent series 1100 HPLC instrument with an Alltech Alltima C₁₈ 5µ, 250 × 4.6 mm, flow: 1 mL/min at 40 °C. Elution was isocratic using a mixture of water, A1 (made up of 700 mL water, 300 mL MeOH, 3 mL Et₃N, and enough phosphoric acid to give a pH of 3.4), and MeOH in a ratio of 15:10:75.

### Example 1(a)

**1-*t*-Butoxycarbonyl-4-morpholin-4-yl-1,2,5,6-tetrahydropyridine (1a).** A flask fitted with a modified Dean-Stark apparatus was charged with 1-*t*-butoxycarbonyl-4-piperidone (25.0 g, 0.125 mol), morpholine (16.4 g, 0.188 mol), *p*-toluenesulfonic acid (0.125 g, 0.60 mmol), and toluene (250 mL). The resultant solution was heated at reflux for 20 hr with the condensate flowing through a bed of molecular sieves on return to the refluxing mixture. The mixture was cooled to 50 °C and concentrated *in vacuo* to an orange oil (39 g) as a mixture of **1a** and excess morpholine. Due to the instability of the compound with even trace amount of water, the mixture was carried on without further purification. ¹H NMR (60 MHz, CDCl₃): δ 4.6 (t, 1H), 4.1-3.5 (m, 8H), 2.9-2.1 (m, 6H), 1.5 (s, 9H).

### Example 2(a)

**1-*t*-Butoxycarbonyl-3-(*N*-methylthiocarbamoyl)-4-morpholin-4-yl-1,2,5,6-tetrahydropyridine (2a)**. To a solution of 1-*t*-butoxycarbonyl-4-morpholin-4-yl-1,2,5,6-tetrahydropyridine (38 g as a crude mixture from above, ~0.125 mol) in CHCl₃ (330 mL) in a flask fitted with a condenser and under an Argon atmosphere, was added methyl isothiocyanate (9.5 g, 0.130 mol). The solution was refluxed 16 hr, and then additional methyl isothiocyanate (9.0 g, 0.123 mol) was added and again refluxed 2 hr to completion. The mixture was concentrated *in vacuo* to an orange oil, then reconcentrated from PhMe (2 × 75 mL) inducing crystallization. The mixture was diluted with hexanes (20 mL), cooled in an ice bath, and the light orange crystals filtered giving 9.0 g (22% for two steps) of **2a**. ¹H NMR (60 MHz, CDCl₃): δ 7.6 (bs, 1H), 4.9-4.2 (m, 2H), 3.8-3.4 (m, 6H), 3.2-2.4 (m, 6H), 3.1 (s [two sets], 3H), 1.4 (s, 9H). MS (APCI): *m*/*z* 342.2 (MH⁺). HPLC analysis showed a purity of greater than 99% with retention time of 4.0 min.

### Example 3

**3-Amino-7-*t*-butoxycarbonyl-4-cyano-2-methyl-(1,2,5,6,7,8-hexahydro-2,7-naphthyridine-1-thione) (3)**. In a flask fitted with a condenser and Ar inlet, 1-N-*t-*butoxycarbonyl-3-(*N*-methylthiocarbamoyl)-4-morpholin-4-yl-1,2,5,6-tetrahydropyridine (7.56 g, 22.1 mmol) and malononitrile (1.46 g, 22.1 mmol) were suspended in EtOH (60 mL). Piperidine (1.88 g, 22.1 mmol) was added and the mixture was brought to reflux. At 50 °C a dark red-brown solution formed. After 5 min at reflux, formation of a bright yellow ppt occurred. The mixture was heated at reflux an additional hr, cooled to rt, and the ppt was filtered. The material was washed with EtOH (5 mL) to give 5.73 g (81 %) of 3 as a bright yellow solid. ¹H NMR (300 MHz, DMSO-d₆): δ 7.7 (s, 2H), 4.3 (s, 2H), 3.9 (s, 3H), 3.5 (t, 2H), 2.6 (t, 2H), 1.4 (s, 9H). MS (APCI): *m*/*z* 289.0 (20, M-NH₂), 305.0 (80, M-NH₂-CH₃). HPLC analysis showed a purity of 97% with retention time of 5.1 min.

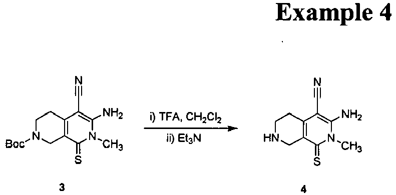

**Preparation of 3-Amino-4-cyano-2-N-methyl-(1,2,5,6,7,8-hexahydro-2,7-naphthyridine-1-thione) (Example 4)** or 3-amino-2-methyl-1-thioxo-1,2,5,6,7,8-hexahydro-2,7-naphthyridine-4-carbonitrile.

To a suspension of 3-Amino-7-N-*t*-butoxycarbonyl-4-cyano-2-N-methyl-(1,2,5,6,7,8-hexahydro-2,7-naphthyridine-1-thione (4.0 g, 12.5 mmol) in CH₂Cl₂ (40 mL) stirring in a flask under Ar, was added trifluoroacetic acid (5.7 g, 50 mmol) forming a red solution. After 1 hr, Et₃N (6.3 g, 63 mmol) was added creating a heavy yellow ppt. The mixture was cooled in an ice bath, filtered, and the solid washed with cold CH₂Cl₂ (2 × 10 mL) giving 2.0 g (73%) of Example 4 as a yellow solid. ¹H NMR (300 MHz, DMSO-d₆): δ 7.6 (bs, 2H), 3.9 (s, 3H), 3.6 (s, 2H), 2.9 (t, 2H), 2.5 (t, 2H). HPLC analysis showed a purity of greater than 98% with retention time of 3.7 min.

### Example 1(b)

**1-N-Methyl-4-morpholin-4-yl-1,2,5,6-tetrahydropyridine (1b).** A flask fitted with a modified Dean-Stark apparatus was charged with 1-methyl-4-piperidone (20.0 g, 0.177 mol), morpholine (21.6 g, 0.248 mol), *p*-toluenesulfonic acid (0.150 g, 0.75 mmol), and toluene (250 mL). The resultant solution was heated at reflux for 20 hr with the condensate flowing through a bed of molecular sieves on return to the refluxing mixture. The mixture was cooled to 50 °C, concentrated *in vacuo* to an orange oil (36 g), and reconcentrated with PhMe (2 × 100 mL) to give 34 g of **1b** as a mixture with some excess morpholine. Due to the instability of the compound with even trace amount of water, the mixture was carried on without further purification. ¹H NMR (60 MHz, CDCl₃): δ 4.6 (t, 1H), 3.9-3.6 (m, 4H), 3.1-2.4 (m, 10H), 2.3 (s, 3H).

### Example 2(b)

### 1-Methyl-3-(N-methylthiocarbamoyl)-4-morpholin-4-yl-1,2,5,6-tetrahydropyridine (2b).

To a solution of 1-methyl-4-morpholin-4-yl-1,2,5,6-tetrahydropyridine (34 g as a crude mixture from above, ~0.177 mol) in CHCl₃ (400 mL) in a flask fitted with a condenser and under Ar atmosphere, was added methyl isothiocyanate (14.6 g, 0.194 mol). The solution was refluxed 16 hr. Analysis of an aliquot by NMR showed only ~10% completion. Additional isothiocyanate (6.0 g, 0.080 mol) was added and the mixture was refluxed 24 hr to ~30% completion by NMR. After another 24 hr, NMR analysis showed the reaction to be ~50% complete. The mixture was concentrated *in vacuo* to an orange oil, then reconcentrated from PhMe (2 × 75 mL) giving a red-orange oil (45 g) as ~1:1 mixture of **1b:2b.** Due to the instability of these compounds to purification, the crude mixture was taken on as is. ¹H NMR (60 MHz, CDCl₃): The protons of the N-methyl group of **2b** grew in as two singlets at 3.1 and 3.2 ppm.

**3-Amino-4-cyano-2,7-dimethyl-(1,2,5,6,7,8-hexahydro-2,7-naphthyridine-1-thione (Example 5)**. In a flask fitted with a condenser and Ar inlet, 1-methyl-3-(*N-*methylthiocarbamoyl)-4-morpholin-4-yl-1,2,5,6-tetrahydropyridine (44.0 g as mixture of ~1:1 with 1b, 172 mmol) and malononitrile (11.4 g, 172 mmol) were dissolved in EtOH (350 mL). Piperidine (14.6 g, 172 mmol) was added and the mixture was brought to reflux. The mixture was heated at reflux 3.5 hr, then cooled to rt, and concentrated *in vacuo* to a dark red oil. The material was partitioned with 1M H₂SO₄ (400 mL) and CH₂Cl₂ (400 mL). The aqueous phase was washed with CH₂Cl₂ (2 × 100 mL) and a ppt started to form. The mixture was stirred for 30 min and the ppt filtered giving 11.7 g of the sulfate salt of Example 5 as an orange-brown solid. The supernatant analyzed by HPLC, contained less than 50% product and numerous impurities and was discarded. The sulfate salt was suspended in H₂O (100 mL) and 1M NaOH was added until a pH of 10 was reached (~35 mL). The tan-orange suspension was filtered giving 9.9 g of crude product as the free base. The material was recystallized from *i*PrOH (250 mL) giving 5.1 g tan crystals, and again with MeOH (100 mL) giving 4.5 g of an off-white solid. The solid was further purified by mixing with Na₂SO₄ (20 g) and filtering through silica gel (40 g) with EtOAc:MeOH 9:1 as an eluent giving 4.3 g (11% for three steps) Example 5 as a light yellow solid. ¹H NMR (300 MHz, DMSO-d₆): δ 7.6 (s, 2H), 3.9 (s, 3H), 3.3 (s, 2H), 2.7 (t, 2H), 2.5 (t, 2H), 2.3 (s, 3H). HPLC analysis showed a purity of greater than 99% with retention time of 4.5 min.

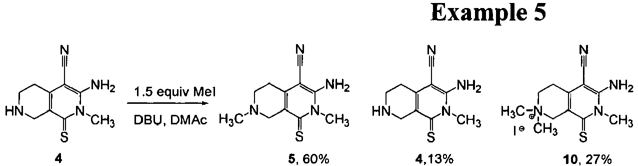

### Preparation of 3-Amino-4-cyano-2,7-dimethyl-(1,2,5,6,7,8-hexahydro-2,7-napthyridine-1-thione) (Example 5), or 3-amino-2,7-dimethyl-1-thioxo-1,2,5,6,7,8-hexahydro-2,7-naphthyridine-4-carbonitrile,

### from 3-Amino-4-cyano-2-methyl-(1,2,5,6,7,8-hexahydro-2,7-naphthyridine-1-thione)

### (Example 4).

To a suspension of 3-Amino-4-cyano-2-methyl(1,2,5,6,7,8-hexahydro-2,7-napthyridine-1-thione) (44 mg, 0.20 mmol) in *N,N*-dimethylacetamide (0.8 mL) was added 1,8-diazabicyclo[5.4.0]undec-7-ene (DBU) (46 mg, 0.30 mmol) followed by MeI (42 mg, 0.30 mmol). The resultant orange solution was stirred 15 min. HPLC analysis showed a ratio of Example 4:Example 5:10 of 13:60:27. The mixture was concentrated under high vacuum to a dark, red oil and purified on silica gel (gradient of EtOAc to EtOAc:MeOH 9:1). The desired product eluted first, followed by starting material.

The three components are also separable by HPLC: Alltech Alltima column 75:15:10 H₂O:A1:MeOH (A1 is made of 700 mL H₂O, 300 mL MeOH, 3 mL Et₃N and sufficient H₃PO₄ to give a pH of 3.4), with retention times of 4.2 min (Example 4), 4.6 min (Example 5), and 4.0 min (10).

Notes: Adding another 0.5 equivalents of MeI gives a ratio of Example 4:Example 5:10 of 2:34:64. The quaternized material (10) precipitates to some degree from the concentrated mixture with EtOH.

The following compounds are prepared as described in the above scheme by substituting the appropriate reactant.

### Example 6

### 2,3-diamino-4-cyano-7-methyl-(1,2,5,6,7,8-hexahydro-2,7-napthyridine-1-thione)

### Example 7

### 2,3-diamino-4-cyano-6,6,7-trimethyl-(1,2,5,6,7,8-hexahydro-2,7-napthyridine-1-thione)

### Example 8

### 3-Amino-2-ethyl-4-cyano-7-methyl-(1,2,5,6,7,8-hexahydro-2,7-napthyridine-1-thione)

### Example 9

### 3-Amino-2-methyl-4-cyano-7-methyl-(1,2,5,6,7,8-hexahydro-2,7-napthyridine-1-ketone)

Of particular interest for the present invention is the compound of Example 5, i.e. 3-Amino-4-cyano-2,7-dimethyl-(1,2,5,6,7,8-hexahydro-2,7-napthyridine-1-thione) or 3-amino-2,7-dimethyl-1-thioxo-1,2,5,6,7,8-hexahydro-2,7-naphthyridine-4-carbonitrile. This compound is shown, below, as a particularly useful tool compound.

The antagonist ability of the compounds of the present invention to block the response of a selective alpha adrenergic receptor agonist is demonstrated in the neuropathic pain model or the Chung rat nerve ligation model (Kim and Chung, 1992, Pain, 50, pp. 355-363). The Chung model is a surgical model of neuropathic pain in rats. The pain state is generated by tight ligation of the L5 and L6 spinal nerves on one side of the rat. The surgical procedure results in a long-lasting mechanical allodynia in the affected foot. Mechanical allodynia is measured using von Frey filaments, where the investigator stimulates the plantar surface of the affected foot. Different sized filaments generate a different force. A painful response is signified by withdrawal of the paw.

For analysis of single compound such as 4-Cyclopent-3-enylmethyl- 1,3-dihydro-imidazole-2-thione or Example 5 in the Chung model, the compound is administered by IP injection at 100 ug/kg at time zero. Mechanical allodynia is measured at 30 minutes post drug and is reported as the percentage response to the baseline reading taken pre-drug administration. For the antagonist study of Example 5, the compounds are co-administered IP at 100 ug/kg each at time zero.

The lack of allodynial reversal in these rats is also a measure of antagonist ability to block a response. The results of using the compound of Example 5 in this model is shown in Figure 1A. It is noted that the compound of Example 5 completely blocks the ability of 4-Cyclopent-3-enylmethyl- 1,3-dihydro-imidazole-2-thione to reverse allodynia in the rat. 4-Cyclopent-3-enylmethyl- 1,3-dihydro-imidazole-2-thione is a selective alpha adrenergic 2B receptor agonist and as determined by the Schild analysis reported below, the compound of Example 5 is a selective alpha adrenergic 2B receptor antagonist.

An important type of pharmacological analysis dedicated to antagonists is the Schild analysis (Schild, H.O., 147, Br. J. Pharmacol., 2,. pp. 189-206). This analysis involves performing concentration response curves for an agonist in the presence of the antagonist of interest. Schild analysis allows determination of whether the antagonist is competitive or not.

### FLIPR Ca⁺² Influx Assay

HEK 293 cells stably expressing the human alpha 2A receptor and the chimeric G protein G_{qi5}, the mouse alpha2B receptor and the G protein G₁₆, and the human alpha 2C receptor and the chimeric G protein G_{qi5} are plated in poly-D-lysine coated 96-well plates at 50,000 - 75,000 cells per well and grown overnight in DMEM supplemented with 10% fetal bovine serum. For FLIPR (fluorometric image plate reader) evaluation, cells are washed twice with HBSS/HEPES Buffer (1X Hanks Buffered Salt Solution, 20 mM HEPES, pH 7.4) prior to the addition of Fluo-4-AM (4 uM Fluo-4-AM, 0.04% pluronic acid in HBSS/HEPES Buffer), a calcium-sensitive dye. Cells are loaded with dye for 60 minutes at 37°C, then washed 4 times with HBSS/HEPES Buffer. For both the agonist and antagonist assay, the test compounds are tested between 0.64 nM - 10,000 nM.

For an agonist assay, the reaction is initiated by the addition of the appropriate dilutions of compounds and the transient calcium signal captured. The peak height of the calcium curve is determined and utilized for calculation of EC₅₀ and efficacy using ActivityBase. Norepinephrine is the standard full agonist used for evaluating alpha-2 receptor activity.

For an antagonist assay, the addition of the drug does not elicit a transient calcium signal. However, the antagonist blocks the transient calcium signal of the standard agonist norepinephrine in a dose-dependent manner. The residual norepinephrine peak height is compared to the non-antagonized norepinephrine peak height for the determination of % antagonism.

### FLIPR Ca⁺² Influx Schild Assay

Compounds that demonstrate antagonism in the standard FLIPR Ca⁺² Influx Assay are further characterized in the Schild Assay which gives a better representation of the strength of the antagonist. In the Schild assay, a range of antagonist concentrations are run against a range of agonists concentrations, to generate a series of dose response curves. As in the standard FLIPR Ca⁺² Influx assay, the test compound, the antagonist, is added first, then challenged with the agonist norepinephrine, which elicits a calcium response. Analysis of the dose response curves results in the generation of a pKB, which is a measure of the affinity of the antagonist for the receptor, and a Hill coefficient, which, when ~ 1.0, designates that the antagonist is reversible and competitive.

This data is reported in Figure 1B.

In Figure 1B the following properties or values for the alpha adrenergic receptor antagonist are reported:
pK_{B}: this is the negative log of K_{B}, which is the equilibrium dissociation constant for the antagonist-receptor complex. It is a term describing the molecular interactions between the antagonist and receptor. The more negative the pK_{B}, the stronger the interaction.
KI: is the inhibition calculation in nM. It is the antilog of the pKB and is a more general way to gauge the antagonist-receptor interaction. A smaller KI indicates better binding.

Hill is the Hill coefficient, which is used to calculate the slope of the antagonism curve. A value at or near 1.0 indicates competitive, reversible (surmountable) antagonism. Competitive, surmountable antagonism occurs when the antagonist and agonist share the same binding site on the receptor and compete for binding to that site.

SEM is the standard error of the mean and is a statistical measure of the data.

R2 is a correlation coefficient for the plot and determines how well the calculated curve fits the data. A number at or near 1.0 signifies good correlation.

## Claims

1. A compound for use in a method of therapy, wherein the compound is 2,3-diamino-6,6,7-trimethyl-1-thioxo-1,2,5,6,7,8-hexahydro-2,7-naphthyridine-4-carbonitrile or the compound is represented by the following formula: 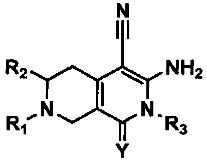 wherein Y is O or S; and R₁ R₂ and R₃ are H, hydrocarbyl, substituted hydrocarbyl or amino.

2. A compound represented by the following formula, for use in a method of therapy: 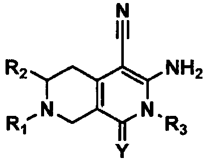 wherein Y is O or S; and wherein R₁, R₂ and R₃ are selected from the group consisting of H, alkyl, aryl and NR₄R₄, wherein R₄ is alkyl.

3. The compound for use according to claim 1, wherein R₁ and R₂ are selected from the group consisting of H and C₁ to C₄ alkyl, and R₃ is selected from the group consisting of H, C₁ to C₄ alkyl and amino.

4. The compound for use according to claim 2, wherein R₁, R₂ and R₃ are selected from the group consisting of H, C₁ to C₄ alkyl, and NR₄R₄, wherein R₄ is C₁ to C₄ alkyl.

5. The compound for use according to claims 1 or 2, wherein the compound is selected from the group consisting of:
3-amino-2,7-dimethyl-1-thioxo-1,2,5,6,7,8-hexahydro-2,7-naphthyridine-4-carbonitrile,
3-amino-2-methyl-1-thioxo-1,2,5,6,7,8-hexahydro-2,7-naphthyridine-4-carbonitrile,
3-amino-2-ethyl-7-methyl-1-thioxo-1,2,5,6,7,8-hexahydro-2,7-naphthyridine-4-carbonitrile, and
3-amino-2,7-dimethyl-1-oxo-1,2,5,6,7,8-hexahydro-2,7-naphthyridine-4-carbonitrile.

6. The compound according to claim 1, wherein the compound is 2,3-diamino-7-methyl-1-thioxo-1,2,5,6,7,8-hexahydro-2,7-naphthyridine-4-carbonitrile,

7. The compound for use according to claim 5, wherein the compound is 3-amino-2,7-dimethyl-1-thioxo-1,2,5,6,7,8-hexahydro-2,7-naphthyridine-4-carbonitrile.

8. The compound for use according to claim 1, wherein Y is S.

9. The compound for use according to claim 2, wherein Y is S.

10. The compound for use according to claim 9, wherein R₁, R₂ and R₃ are selected from the group consisting of H, C₁ to C₄ alkyl, and NR₄R₄, wherein R₄ is C₁ to C₄ alkyl.

11. The compound for use according to claims 3, 4 or 10 wherein the C₁ to C₄ alkyl is methyl, ethyl, propyl, isopropyl, butyl, isobutyl or tertiary butyl.

12. The compound for use according to claim 1, wherein the compound is

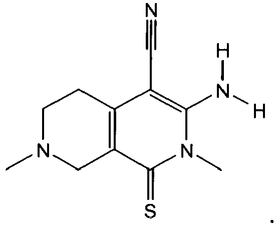

13. The compound for use according to any one of claims 1 to 12, wherein the method of therapy is a method of treating pain, glaucoma, ischemic neuropathy, neurodegenerative disease, myocardial infarction or a method of preventing acute coronary events.

14. The compound for use according to any one of claims 1 to 13, wherein the method of therapy is a method of treating chronic pain, visceral pain, corneal pain, or neuropathic pain.

## Patentansprüche

1. Verbindung zur Verwendung in einem Therapieverfahren, wobei die Verbindung 2,3-Diamino-6,6,7-trimethyl-1-thioxo-1,2,5,6,7,8-hexahydro-2,7-naphthyridin-4-carbonitril oder eine Verbindung ist, die durch die folgende Formel dargestellt wird: 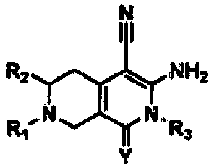 worin Y O oder S ist; und R₁, R₂ und R₃ H, Hydrocarbyl, substituiertes Hydrocarbyl oder Amino sind.

2. Verbindung, dargestellt durch die folgende Formel zur Verwendung in einem Therapieverfahren: 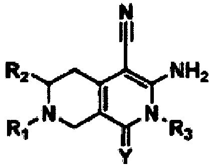 worin Y O oder S ist; und worin R₁, R₂ und R₃ ausgewählt sind aus der Gruppe, bestehend aus H, Alkyl, Aryl und NR₄R₄, worin R₄ Alkyl ist.

3. Verbindung zur Verwendung gemäß Anspruch 1, worin R₁ und R₂ ausgewählt sind aus der Gruppe, bestehend aus H und C₁₋₄-Alkyl, und R₃ ausgewählt ist aus der Gruppe, bestehend aus H, C₁₋₄-Alkyl und Amino.

4. Verbindung zur Verwendung gemäß Anspruch 2, worin R₁, R₂ und R₃ ausgewählt sind aus der Gruppe, bestehend aus H, C₁₋₄-Alkyl und NR₄R₄, worin R₄ C₁₋₄-Alkyl ist.

5. Verbindung zur Verwendung gemäß Anspruch 1 oder 2, worin die Verbindung ausgewählt ist aus der Gruppe, bestehend aus:
3-Amino-2,7-dimethyl-1-thioxo-1,2,5,6,7,8-hexahydro-2,7-naphthyridin-4-carbonitril,
3-Amino-2-methyl-1-thioxo-1,2,5,6,7,8-hexahydro-2,7-naphthyridin-4-carbonitril,
3-Amino-2-ethyl-7-methyl-1-thioxo-1,2,5,6,7,8-hexahydro-2,7-naphthyridin-4-carbonitril und
3-Amino-2,7-dimethyl-1-oxo-1,2,5,6,7,8-hexahydro-2,7-naphthyridin-4-carbonitril.

6. Verbindung gemäß Anspruch 1, worin die Verbindung 2,3-Diamino-7-methyl-1-thioxo-1,2,5,6,7,8-hexahydro-2,7-naphthyridin-4-carbonitril ist.

7. Verbindung zur Verwendung gemäß Anspruch 5, worin die Verbindung 3-Amino-2,7-dimethyl-1-thioxo-1,2,5,6,7,8-hexahydro-2,7-naphthyridin-4-carbonitril ist.

8. Verbindung zur Verwendung gemäß Anspruch 1, worin Y S ist.

9. Verbindung zur Verwendung gemäß Anspruch 2, worin Y S ist.

10. Verbindung zur Verwendung gemäß Anspruch 9, worin R₁, R₂ und R₃ ausgewählt sind aus der Gruppe, bestehend aus H, C₁₋₄-Alkyl und NR₄R₄, worin R₄ C₁₋₄-Alkyl ist.

11. Verbindung zur Verwendung gemäß Ansprüche 3, 4 oder 10, worin das C₁₋₄-Alkyl Methyl, Ethyl, Propyl, Isopropyl, Butyl, Isobutyl oder tertiäres Butyl ist.

12. Verbindung zur Verwendung gemäß Anspruch 1, worin die Verbindung 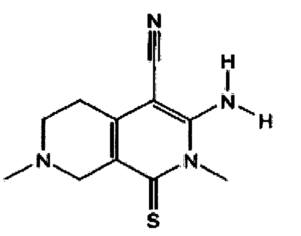 ist.

13. Verbindung zur Verwendung gemäß einem der Ansprüche 1 bis 12, worin das Therapieverfahren ein Verfahren zur Behandlung von Schmerzen, Glaukom, ischämischer Neuropathie, neurodegenerativer Erkrankung, Herzinfarkt oder ein Verfahren zur Verhinderung akuter koronarer Ereignisse ist.

14. Verbindung zur Verwendung gemäß einem der Ansprüche 1 bis 13, worin das Therapieverfahren ein Verfahren zur Behandlung chronischer Schmerzen, viszeraler Schmerzen, kornealer Schmerzen oder neuropathischer Schmerzen ist.

## Revendications

1. Composé pour utilisation dans un procédé de thérapie, dans lequel le composé est le 2,3-diamino-6,6,7-triméthyl-1-thioxo-1,2,5,6,7,8-hexahydro-2,7-naphtyridine-4-carbonitrile ou le composé est représenté par la formule suivante : 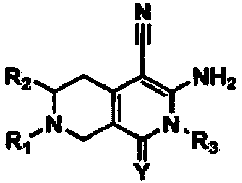 dans laquelle Y est O ou S ; et R₁, R₂ et R₃ sont H, un hydrocarbyle, un hydrocarbyle substitué ou un amino.

2. Composé représenté par la formule suivante pour utilisation dans un procédé de thérapie : 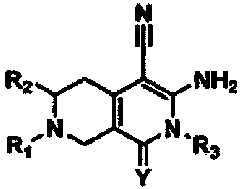 dans laquelle Y est O ou S ; et dans laquelle R₁, R₂ et R₃ sont choisis dans le groupe constitué par H, un alkyle, un aryle et NR₄R₄, où R₄ est un alkyle.

3. Composé pour utilisation selon la revendication 1, dans lequel R₁ et R₂ sont choisis dans le groupe constitué par H et un alkyle en C₁ à C₄, et R₃ est choisi dans le groupe constitué par H, un alkyle en C₁ à C₄ et un amino.

4. Composé pour utilisation selon la revendication 2, dans lequel R₁, R₂ et R₃ sont choisis dans le groupe constitué par H, un alkyle en C₁ à C₄ et NR₄R₄, où R₄ est un alkyle en C₁ à C₄.

5. Composé pour utilisation selon la revendication 1 ou 2, dans lequel le composé est choisi dans le groupe constitué par :
le 3-amino-2,7-diméthyl-1-thioxo-1,2,5,6,7,8-hexahydro-2,7-naphtyridine-4-carbonitrile,
le 3-amino-2-méthyl-1-thioxo-1,2,5,6,7,8-hexahydro-2,7-naphtyridine-4-carbonitrile,
le 3-amino-2-éthyl-7-méthyl-1-thioxo-1,2,5,6,7,8-hexahydro-2,7-naphtyridine-4-carbonitrile et
le 3-amino-2,7-diméthyl-1-oxo-1,2,5,6,7,8-hexahydro-2,7-naphtyridine-4-carbonitrile.

6. Composé selon la revendication 1, dans lequel le composé est le 2,3-diamino-7-méthyl-1-thioxo-1,2,5,6,7,8-hexahydro-2,7-naphtyridine-4-carbonitrile.

7. Composé pour utilisation selon la revendication 5, dans lequel le composé est le 3-amino-2,7-diméthyl-1-thioxo-1,2,5,6,7,8-hexahydro-2,7-naphtyridine-4-carbonitrile.

8. Composé pour utilisation selon la revendication 1, dans lequel Y est S.

9. Composé pour utilisation selon la revendication 2, dans lequel Y est S.

10. Composé pour utilisation selon la revendication 9, dans lequel R₁, R₂ et R₃ sont choisis dans le groupe constitué par H, un alkyle en C₁ à C₄ et NR₄R₄, où R₄ est un alkyle en C₁ à C₄.

11. Composé pour utilisation selon la revendication 3, 4 ou 10, dans lequel l'alkyle en C₁ à C₄ est un méthyle, un éthyle, un propyle, un isopropyle, un butyle, un isobutyle ou un tert-butyle.

12. Composé pour utilisation selon la revendication 1, dans lequel le composé est le suivant :

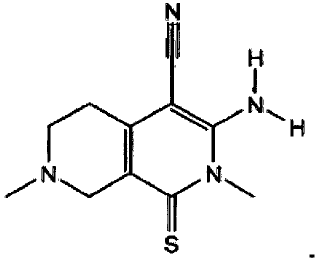

13. Composé pour utilisation selon l'une quelconque des revendications 1 à 12, dans lequel le procédé de thérapie est un procédé de traitement de la douleur, d'un glaucome, d'une neuropathie ischémique, d'une maladie neurodégénérative, de l'infarctus du myocarde ou un procédé de prévention d'événements coronariens aigus.

14. Composé pour utilisation selon l'une quelconque des revendications 1 à 13, dans lequel le procédé de thérapie est un procédé de traitement d'une douleur chronique, d'une douleur viscérale, d'une douleur de la cornée ou d'une douleur neuropathique.
